Europäisches Patentamt

European Patent Office

Office européen des brevets

(11) Veröffentlichungsnummer: **0 412 202 A1**

(12) **EUROPÄISCHE PATENTANMELDUNG**

(21) Anmeldenummer: 89114925.4

(22) Anmeldetag: 11.08.89

(51) Int. Cl.⁵: **A61B 17/22**, G10K 11/28

(43) Veröffentlichungstag der Anmeldung:
13.02.91 Patentblatt 91/07

(84) Benannte Vertragsstaaten:
**DE FR GB NL**

(71) Anmelder: **Siemens Aktiengesellschaft**
**Wittelsbacherplatz 2**
**D-8000 München 2(DE)**

(72) Erfinder: **Hassler, Dietrich, Dipl.-Ing.**
**Flurweg 3**
**D-8525 Uttenreuth(DE)**
Erfinder: **Reichenberger, Helmut, Dipl.-Phys,**
**Dr.rer.nat.**
**Begonienstrasse 28**
**D-8501 Eckental(DE)**
Erfinder: **Schwark, Hubert**
**Schobertweg 20**
**D-8520 Erlangen(DE)**
Erfinder: **Schmidt, Erhard, Ing.grad.**
**Heuwaagstrasse 20**
**D-8520 Erlangen(DE)**

(54) Stosswellenquelle zur Erzeugung von fokussierten Stosswellen mit einem als Rotationsparaboloid ausgebildeten Reflektor.

(57) Die Erfindung betrifft eine Stoßwellenquelle zur Erzeugung von fokussierten Stoßwellen, aufweisend eine im wesentlichen hohlzylindrische Membran (1) aus einem elektrisch leitenden Werkstoff, eine innerhalb der Membran (1) angeordnete elektrische Spulenanordnung, welche zum stoßartigen Antrieb der Membran (1) mit einem Hochspannungsimpuls beaufschlagbar ist, einen die Membran (1) umgebenden konkaven Reflektor (33) in Form eines Rotationsparaboloids, dessen Mittelachse im wesentlichen der Mittelachse der Membran (1) entspricht, und ein wenigstens den Raum zwischen der Membran (1) und dem Reflektor (33) ausfüllendes akustisches Ausbreitungsmedium (37). Dabei ist vorgesehen, daß die Spulenanordnung wenigstens eine zylinderschraubenförmig gewickelte Spule (2) aufweist, und daß die Membran (1) als dünnwandiges, glattes Rohr ausgebildet ist.

FIG 1

EP 0 412 202 A1

## STOSSWELLENQUELLE ZUR ERZEUGUNG VON FOKUSSIERTEN STOSSWELLEN MIT EINEM ALS ROTATIONSPARABOLOID AUSGEBILDETEN REFLEKTOR

Die Erfindung betrifft eine Stoßwellenquelle zur Erzeugung von fokussierten Stoßwellen, aufweisend eine im wesentlichen hohlzylindrische Membran aus einem elektrisch leitenden Werkstoff, eine innerhalb der Membran angeordnete elektrische Spulenanordnung, welche zum stoßartigen Antrieb der Membran mit einem Hochspannungsimpuls beaufschlagbar ist, einen die Membran umgebenden konkaven Reflektor, dessen Form wenigstens näherungsweise die eines Rotationsparaboloids ist und dessen Mittelachse im wesentlichen der Mittelachse der Membran entspricht, und ein wenigstens den Raum zwischen der Membran und dem Reflektor ausfüllendes akustisches Ausbreitungsmedium.

Derartige elektromagnetische Stoßwellenquellen können für die unterschiedlichsten Zwecke verwendet werden, z.B. in der Medizin, um im Körper eines Patienten befindliche Konkremente nicht-invasiv zu zertrümmern oder pathologische Geweberänderungen ebenfalls nicht-invasiv zu behandeln. Außerdem können derartige Stoßwellenquellen in der Werkstoffprüfung eingesetzt werden, um Materialproben mit fokussierten Stoßwellen zu beaufschlagen. Dabei wird die Stoßwellenquelle in geeigneter Weise mit dem jeweils zu beschallenden Objekt akustisch gekoppelt, so daß die erzeugten Stoßwellen in das Objekt eingeleitet werden können. Die Funktion derartiger Stoßwellenquellen beruht im wesentlichen darauf, daß die Membran bei Beaufschlagung der Spulenanordnung mit einem Hochspannungsimpuls radial expandiert, wodurch in das Ausbreitungsmedium ein Druckimpuls in Gestalt einer Zylinderwelle eingeleitet wird, der sich allmählich zu einer Stoßwelle aufsteilt. Der Druckimpuls bzw. die Stoßwelle wird an dem Reflektor in der Weise reflektiert, daß die akustische Energie in dem Brennpunkt des Rotationsparaboloids konzentriert wird. Die Stoßwellenquelle und das zu beschallende Objekt müssen relativ zueinander so ausgerichtet sein, daß der zu beschallende Bereich des Objektes sich in dem Brennpunkt des Rotationsparaboloids befindet, der dem Zentrum des Fokusbereiches der Stoßwellen entspricht.

Stoßwellenquellen der eingangs genannten Art sind unter der Bezeichnung "Large Aperture Ringshaped Soundsource" (LARS) bekannt geworden. Im Gegensatz zu anderen bekannten elektromagnetischen Stoßwellenquellen, bei denen zunächst ebene Stoßwellen erzeugt werden, die dann mittels geeigneter akustischer Linsen fokussiert werden (DE-OS 33 28 039), oder bei denen eine kugelkalottenförmig geformte Membran vorgesehen ist, von der Stoßwellen ausgehen, die keiner weiteren Fokussierung bedürfen (DE-OS 34 43 295), werden im Falle der Stoßwellenquelle der eingangs genannten Art zunächst Zylinderwellen erzeugt, die dann durch Reflexion an dem rotationsparaboloidförmigen Reflektor in einem Fokus konzentriert werden. Bei einer bekannten Stoßwellenquelle der eingangs genannten Art weist die Spulenanordnung mehrere Flachspulen auf, die nebeneinanderliegend in einer zylinderförmig gekrümmten Fläche innerhalb der hohlzylindrischen Membran angeordnet sind. Die Herstellung dieser Spulenanordnung ist mit einem ganz erheblichen Aufwand verbunden und zieht daher hohe Kosten nach sich. Außerdem wird der für die Spulenanordnung zur Verfügung stehende Raum nur unvollständig ausgenutzt, was sich dadurch bemerkbar macht, daß der Wirkungsgrad der bekannten Stoßwellenquelle deutlich unter dem theoretisch möglichen Wirkungsgrad liegt. Es kommt hinzu, daß infolge der beschriebenen Ausbildung der Spulenanordnung kein gleichmäßiger Antrieb der Membran entlang ihres Umfanges möglich ist. Dies bedeutet, daß die Membran entlang ihres Umfanges lokal unterschiedlichen Verformungen unterworfen ist, und deshalb ungünstigen mechanischen Beanspruchungen ausgesetzt ist, die zu einem vorzeitigen Ausfall der Membran führen können. Es besteht zwar die Möglichkeit, die Membran mit Sicken zu versehen, die in denjenigen Bereichen vorgesehen sind, in denen benachbarte Spulen der Spulenanordnung aneinandergrenzen, so daß sich die Membran unter der Wirkung der Antriebskräfte leichter verformen kann, jedoch verteuert diese Maßnahme die Herstellung der Membran erheblich, ganz abgesehen davon, daß mit dieser Maßnahme eine Verschlechterung der Fokussierungswirkung verbunden ist, da von einer in der beschriebenen Weise mit Sicken versehenen Membran keine idealen Zylinderwellen ausgehen können.

Der Erfindung liegt die Aufgabe zugrunde, eine Stoßwellenquelle der eingangs genannten Art so auszubilden, daß sie mit geringem Fertigungsaufwand und geringen Kosten herstellbar ist, daß ein gleichmäßiger Antrieb der Membran gewährleistet ist und daß ein hoher Wirkungsgrad erzielt wird.

Nach der Erfindung wird diese Aufgabe dadurch gelöst, daß die Spulenanordnung wenigstens eine zylinderschraubenförmig gewickelte Spule aufweist und daß die Membran als dünnwandiges, glattes Rohr ausgebildet ist. Infolge des Umstandes, daß die Spule zylinderschraubenförmig gewickelt ist, wird zunächst erreicht, daß der Herstellungsaufwand für die Spulenanordnung denkbar gering ist. Zugleich wird durch diese Ausbildung der Spule eine optimale Nutzung des für die Spulenanordnung zur Verfügung stehenden Raumes ermöglicht, so daß ein hoher Wirkungsgrad erzielt wird. Weiter wird durch diese Ausbildung der Spule ein gleichmäßiger Antrieb der Membran sichergestellt, so daß diese durch die Antriebskräfte einer gleichmäßigen mechanischen Beanspruchung unterworfen ist. Infolge dieses Umstandes und infolge des Umstandes, daß die Membran als dünnwandiges, glattes Rohr ausgebildet ist, ist gewährleistet, daß von der Membran tatsächlich ideale Zylinderwellen ausgehen, was für eine gute Fokussierungswirkung unerläßlich ist. Schließlich ist die Membran infolge ihrer Ausbildung als dünnwandiges, glattes Rohr mit minimalem Aufwand herstellbar. Die Vorteile der Erfindung kommen verstärkt zum Tragen, wenn die Spule gemäß einer bevorzugten Variante der Erfindung konzentrisch zur Membran angeordnet ist, da dann besonders gleichmäßige Antriebs- und Beanspruchungsverhältnisse für die Membran vorliegen.

Gemäß einer Variante der Erfindung ist vorgesehen, daß die Membran als nahtloses Rohr ausgeführt ist. Durch diese Maßnahme wird eine hohe Standzeit der Membran gewährleistet, da eine Naht, die notwendigerweise eine mechanische Schwachstelle darstellen muß, vermieden ist. Um die Abstrahlung idealer Zylinderwellen mittels der Membran weiter zu begünstigen, weist diese vorzugsweise eine konstante Dicke auf. Die Membran enthält vorzugsweise wenigstens ein Material der Gruppe Aluminium, Kupfer, Silber oder gut leitender Legierungen derselben, wie z.B. Bronzen. Dabei ist als Material für die Membran Aluminium zu bevorzugen, da dieses Material den Vorteil hoher elektrischer Leitfähigkeit bei guter mechanischer Festigkeit und Bearbeitbarkeit aufweist.

Es versteht sich, daß die Windungen der Spule eine geeignete Isolierung aufweisen müssen, um eine ausreichende elektrische Spannungsfestigkeit der Spule zu gewährleisten. Ebenfalls im Interesse einer ausreichenden Spannungsfestigkeit ist gemäß einer Ausführungsform der Erfindung vorgesehen, daß zwischen der Spule und der Innenwand der Membran Isoliermittel vorgesehen sind. Es kann sich hierbei um eine den Draht der Spule umgebende Isolation handeln, sofern diese eine ausreichende Isolierwirkung besitzt. Vorzugsweise sind die Isoliermittel jedoch durch eine zwischen der Innenwand der Membran und der Spule angeordnete Isolierfolie gebildet.

Gemäß einer bevorzugten Ausführrugnsform der Erfindung ist vorgesehen, daß der zwischen der Innenwand der Membran und der Spule bzw. den Isoliermitteln befindliche Raum mit Unterdruck beaufschlagbar ist. Hierdurch wird zunächst erreicht, daß die Membran sich vor der Erzeugung einer Stoßwelle möglichst dicht bei der Spule befindet, was sich auf den Wirkungsgrad der Stoßwellenquelle positiv auswirkt. Außerdem wird durch diese Maßnahme gewährleistet, daß die Membran nach der Erzeugung einer Stoßwelle wieder in eine definierte Ausgangslage zurückgeführt wird, so daß aufeinanderfolgend erzeugte Stoßwellen die gleichen Charakteristika aufweisen.

Eine weitere bevorzugte Ausführungsform sieht vor, daß im Inneren der Spule eine Ultraschall-Ortungseinrichtung angeordnet ist, mittels derer der Fokusbereich der Stoßwellen abtastbar ist. Diese Ausführungsform ist insbesondere dann von Bedeutung, wenn die Stoßwellenquelle für medizinische Zwecke, z.B. zur Zertrümmerung von Konkrementen im Körper eines Patienten, eingesetzt werden soll, wobei dann die Stoßwellenquelle mit Hilfe der Ultraschall-Ortungseinrichtung relativ zu dem Körper des Patienten derart ausgerichtet wird, daß sich das Zielgebiet, z.B. das zu zertrümmernde Konkrement, im Foksübereich der Stoßwellen befindet. Ein besonderer Vorteil dieser Ausführungsform liegt darin, daß der im Inneren der Spule ohnehin vorhandene Raum zur Aufnahme der Ultraschall-Ortungseinrichtung genutzt wird. Es sind also keine besonderen konstruktiven Maßnahmen erforderlich, um die Ultraschall-Ortungseinrichtung in die Stoßwellenquelle integrieren zu können.

Eine weitere Variante der Erfindung sieht vor, daß die Spule auf einen wenigstens im Bereich der Spule zylindrisch ausgebildeten Spulenträger aus einem elektrisch isolierenden Werkstoff gewickelt ist. Durch diese Maßnahme wird auf einfache Weise eine mechanisch stabile Halterung der Spule erreicht. Dabei kann der Spulenträger in besonders vorteilhafter Weise eine zentrale Bohrung aufweisen, in der die Ultraschall-Ortungseinrichtung angeordnet ist.

Wenn der Reflektor wenigstens im Bereich seiner Reflektorfläche aus einem Material, z.B. Messing, gebildet ist, das akustisch härter als das Ausbreitungsmedium, z.B. Wasser, ist, strahlt die erfindungsgemäße Stoßwellenquelle Stoßwellen in Form von Druckimpulsen ab und wird daher bevorzugt zur Behandlung eines Patienten mit fokussierten Stoßwellen verwendet, und zwar insbesondere zur Behandlung von Steinleiden, Knochenerkrankungen, aber auch zur Behandlung von pathologischen Gewebeveränderungen. Wenn der Reflektor gemäß einer Variante der Erfindung wenigstens im Bereich seiner Reflektorfläche aus einem Material, vorzugsweise aus einem Schaumstoff mit geschlossenen Poren, gebildet ist, das akustisch weicher als das Ausbrei tungsmedium, z.B. Wasser, ist, werden Stoßwellen in Form von Unterdruckimpulsen erzeugt. Die Stoßwellenquelle kann dann insbesondere zur Behandlung von pathologischen Gewebeveränderungen, z.B. Tumorleiden, Verwendung finden, da Unterdruck die Ausbildung von Kavitation fördert, welche in tierexperimentellen Untersuchungen zu einer Schädigung von Zellen und Gewebe geführt hat.

Ausführungsbeispiele der Erfindung sind in den Figuren der beigefügten Zeichnungen dargestellt. Es zeigen:

Fig. 1 eine vorzugsweise zur Zertrümmerung von Konkrementen im Körper eines Patienten vorgesehene erfindungsgemäße Stoßwellenquelle in schematischer Darstellung im Längsschnitt, und

Fig. 2 eine zur Behandlung von Tumorleiden vorgesehene erfinfindungsgemäße Stoßwellenquelle in schematischer Darstellung im Längsschnitt.

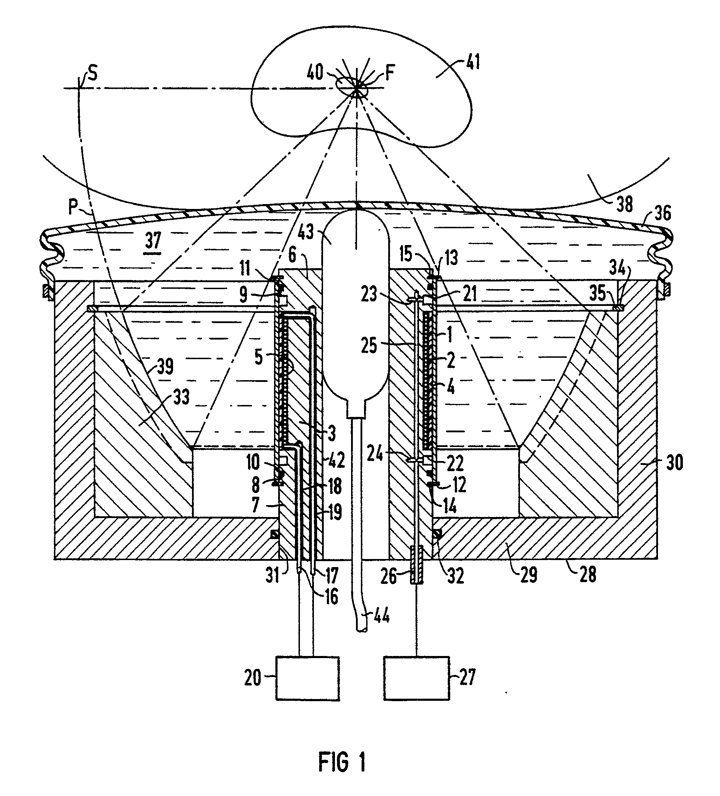

Die in Fig. 1 dargestellte Stoßwellenquelle besitzt eine im wesentlichen hohlzylindrische Membran 1 aus einem elektrisch leitenden Werkstoff, die als dünnwandiges, glattes, nahtloses Rohr konstanter Dicke ausgeführt ist. Die Membran 1 enthält wenigstens ein Material der Gruppe Aluminium, Kupfer, Silber oder deren Legierungen. Innerhalb der Membran 1 befindet sich eine Spule 2, die zylinderschraubenförmig auf einen zylindrischen Spulenträger 3 aus einem elektrisch isolierenden Werkstoff gewickelt ist. Die Spule 2 ist zur Vermeidung von Kurzschlüssen bzw. Spannungsüberschlägen zwischen ihren Windungen aus einem mit einer Lackisolierung versehenen Draht, vorzugsweise Kupferlackdraht, gewickelt. Die Windungen der Spule sind in nicht dargestellter Weise mittels eines geeigneten Tauch-bzw. Vergußharzes auf dem Spulenträger 3 fixiert. Um Spannungsüberschläge zwischen der Spule 2 und der Membran 1 zu vermeiden, die Spule 2 wird zur Erzeugung von Stoßwellen mit Hochspannungsimpulsen hoher Stromstärke beaufschlagt, ist die Spule 2 an ihrer äußeren Mantelfläche vollständig von einer Isolierfolie 4 umgeben, deren Dicke in der Fig. 1 wie auch die Dicke der Membran 1 und der Spule 2 übertrieben dargestellt ist.

Der Spulenträger 3 weist an seiner äußeren Mantelfläche eine ringförmige Eindrehung 5 auf, in der die Spule 2 und die Isolierfolie 4 aufgenommen sind. Die Länge der Eindrehung 5 ist gleich der Länge der Spule 2. Die Tiefe der Eindrehung 5 ist gleich der Summe der Dicken der Spule 2 und der Isolierfolie 4 oder geringfügig größer als diese Summe. Der Spulenträger 3 weist also beiderseits der Spule 2 je einen zylindrischen Ansatz 6, 7 auf, wobei der Durchmesser der Ansätze 6, 7 im wesentlichen gleich dem Innendurchmesser der Membran 1 ist. Diese ist unter Zwischenfügung von in entsprechenden in den Ansätzen 6, 7 vorgesehenen Nuten 8, 9 aufgenommenen Dichtringen 10, 11 auf den Spulenträger 3 aufgesetzt. Mit Hilfe von Sprengringen 12, 13, die an den Enden der Membran 1 anliegen und in entsprechenden Nuten 14, 15 der Ansätze 6, 7 des Spulenträgers 3 aufgenommen sind, ist die Membran 1 auf dem Spulenträger 3 axial unverschieblich fixiert.

Die Anschlüsse 16, 17 der Spule sind durch in dem Spulenträger 3 vorgesehene abgewinkelte Bohrungen 18, 19 zur Stirnfläche des Ansatzes 7 des Spulenträgers 3 geführt und mit einem schematisch angedeuteten Hochspannungs-Impulsgenerator 20 verbunden. Zwischen der Eindrehung 5 des Spulenträgers 3 und den die Dichtringe 10, 11 aufnehmenden Nuten 8, 9 ist jeweils eine weitere Nut 21, 22 vorgesehen. Die Nuten 21, 22 stehen über in dem Spulenträger 3 etwa radial verlaufende Bohrungen 23, 24 mit einer etwa axial in dem Spulenkörper 3 verlaufenden Bohrung 25 in Verbindung, welche im Bereich der Stirnfläche des Ansatzes 7 des Spulenträgers 3 endet und mittels einer Leitung 26 an eine in Fig. 1 schematisch angedeutete Vakuumpumpe 27 angeschlossen ist. Es ist so möglich, den zwischen der Innenwand der Membran 1 und der Isolierfolie 4 befindlichen Raum mit Unterdruck zu beaufschlagen.

Außerdem besitzt die Stoßwellenquelle gemäß Fig. 1 ein topfförmiges Gehäuse 28 mit einem Boden 29 und einer zylinderrohrförmigen Wand 30. Der Spulenträger 3 ist mit seinem Ansatz 7 in einer Bohrung 31 des Bodens 29 des Gehäuses 28 unter Zwischenfügung eines Dichtringes 32 derart fixiert, daß die gemeinsame Mittelachse der Membran 1 und der Spule 2 mit der Mittelachse des Gehäuses 28 zusammenfällt.

In dem Gehäuse 28 ist ein ringförmiger Reflektor 33 aufgenommen, dessen äußere Mantelfläche an der Innenseite der Wand 30 des Gehäuses 28 anliegt. Der Reflektor 33 liegt mit seiner einen Stirnfläche an dem Boden 29 des Gehäuses 28 an und ist mittels eines in einer entsprechenden in der Wand 30 des Gehäuses 28 vorgesehenen Nut 34 aufgenommenen Sprengringes 35 axial unverschieblich in dem Gehäuse 28 fixiert.

An seinem offenen Ende ist das Gehäuse 28 mittels eines flexiblen Balges 36 verschlossen. Der gesamte von dem Balg 36, dem Gehäuse 28, dem Reflektor 33, dem Spulenträger 3 und der Membran 1 umschlossene Raum ist mit einem akustischen Ausbreitungsmedium 37, z.B. Wasser, gefüllt. Mittels des Balges 36 ist es möglich, die Stoßwellenquelle akustisch mit dem in Fig. 1 schematisch angedeuteten Körper 38 eines Patienten zu koppeln, indem die Stoßwellenquelle wie dargestellt mittels des Balges 36 an den Körper des Patienten angepreßt wird.

Der die Membran 1 umgebende ringförmige Reflektor 33 besitzt die Form eines Rotationsparaboloids, dessen Mittelachse mit der der Membran 1 und damit auch der der Spule 2 zusammenfällt. In anderen Worten ausgedrückt besitzt der Reflektor 33 eine konkave Reflektorfläche 39, die durch Rotation eines Abschnittes einer in Fig. 1 strichpunktiert angedeuteten Parabel P um die Mittelachse der Membran 1 erhalten wird, wobei der Brennpunkt F der Parabel P auf der Mittelachse der Membran 1 und der Scheitelpunkt S der Parabel P auf einer die Mittelachse der Membran 1 rechtwinklig schneidenden Geraden liegt. Dabei ist zu berücksichtigen, daß der Brennpunkt F der Parabel P im Betrieb der Stoßwellenquelle, wie noch erläutert werden wird, dem Zentrum des Fokusbereiches der erzeugten Stoßwellen entspricht. In der Regel ist die Erstreckung der Reflektorfläche 39 in Richtung der Mittelachse der Membran 1, wie in Fig. 1 dargestellt, gleich der Länge der Spule 2. In diesem Falle ist wie bei dem in Fig. 1 dargestellten Ausführungsbeispiel die Reflektorfläche 39 in axialer Richtung relativ zu der Spule 2 derart angeordnet ist, daß die einander entsprechenden Enden der Reflektorfläche 39 und der Spule 2 einander radial gegenüberliegen. Es ist jedoch grundsätzlich auch möglich, die Reflektorfläche 39 mit einer von der der Spule 2 abweichenden Erstreckung in Richtung der Mittelachse der Membran auszuführen und/oder die Reflektorfläche 39 relativ zu der Spule 2 axial versetzt anzuordnen. Der Reflektor 33 besteht aus einem Material, das akustisch härter als das Ausbreitungsmedium 37 ist. Wenn als Ausbreitungsmedium 37 Wasser vorgesehen ist, kommt als Material für den Reflektor 33 ein metallischer Werkstoff, beispielsweise Messing, in Frage. Dabei genügt es, wenn das genannte Material wie strichliert angedeutet wenigstens im Bereich der Reflektorfläche 39 des Reflektors 33 als ausreichend dicke Schicht vorhanden ist. Im Falle des Ausführungsbeispieles besteht der Reflektor 33 jedoch insgesamt aus dem genannten Material.

Die Funktionsweise der Stoßwellenquelle gemäß Fig. 1 ist folgende:

Wird die Spule 2 mittels des Hochspannungs-Impulsgenerators 20 mit einem Hochspannungsimpuls beaufschlagt, hat dies zur Folge, daß die Spule 2 äußerst schnell ein magnetisches Feld aufbaut. Hierdurch wird gleichzeitig in die Membran 1 ein Strom induziert, der dem in der Spule 2 fließenden Strom entgegengerichtet ist und demzufolge ein magnetisches Gegenfeld erzeugt, unter dessen Wirkung die Membran 1 schlagartig radial expandiert. Hierdurch wird von der Membran 1 in dem im Inneren der Stoßwellenquelle befindlichen Ausbreitungsmedium 37 ein sich radial auswärts ausbreitender Druckimpuls in der Gestalt einer Zylinderwelle erzeugt, der, so wie dies für die "Randstrahlen" der Zylinderwelle in Fig. 1 strichliert angedeutet ist, an der rotationsparaboloidförmigen Reflektorfläche des Reflektors 33 derart reflektiert wird, daß er in dem Brennpunkt F der Parabel P zusammenläuft. Auf seinem Ausbreitungsweg geht der Druckimpuls, der sich infolge der nicht-linearen Kompressionseigenschaften des Ausbreitungsmediums 37 allmählich aufsteilt, in eine Stoßwelle über. Es wird also deutlich, daß die Stoßwellenquelle gemäß Fig. 1 Stoßwellen erzeugt, die in einem Fokusbereich zusammenlaufen, dessen Zentrum dem Brennpunkt F der Parabel P entspricht. Bei den so erzeugten Stoßwellen handelt es sich infolge des Umstandes, daß der Reflektor 33 aus einem Material gebildet ist, das akustisch härter als das Ausbreitungsmedium 37 ist, - unter einer größeren akustischen Härte versteht man, daß der akustische Wellenwiderstand dieses Materials größer als der des Ausbreitungsmediums ist, - um Stoßwellen in Form von Druckimpulsen.

Während der Erzeugung von Stoßwellen wird der zwischen der Membran 1 und der Isolierfolie 4 befindliche Raum über die Leitung 26 und die Bohrungen 23, 24, 25 mittels der Vakuumpumpe 27 mit Unterdruck beaufschlagt, um einerseits im Interesse eines hohen Wirkungsgrades der Stoßwellenquelle sicherzustellen, daß sich die Membran 1 vor Beaufschlagung der Spule 2 mit einem Hochspannungsimpuls möglichst nahe bei der Spule 2 befindet, und andererseits zu gewährleisten, daß die Membran 1 nach erfolgter Abgabe eines Druckimpulses in eine definierte Ausgangslage zurückkehrt.

Um im Körper 38 eines Patienten befindliche Konkremente, in Fig. 1 ist schematisch der Stein 40 einer Niere 41 angedeutet, zertrümmern zu können, wird die Stoßwellenquelle mittels des flexiblen Balges 36 an die Körperoberfläche des Patienten zur akustischen Koppelung angepreßte Stoßwellenquelle derart ausgerichtet, daß sich das zu zertrümmernde Konkrement 40, so wie dies in Fig. 1 dargestellt ist, im Brennpunkt F der Parabel P und damit im Fokusbereich der Stoßwellen befindet. Unter der Wirkung einer Anzahl von aufeinanderfolgend erzeugter Stoßwel len zerfällt das Konkrement 40 dann in Bruchstücke, die auf natürlichem Wege abgehen können.

Um die Stoßwellenquelle in der beschriebenen Weise relativ zu dem Körper 38 des Patienten ausrichten zu können, ist in einer zentralen Bohrung 42 des Spulenträgers 3 eine in Fig. 1 nur schematisch angedeutete Ultraschall-Ortungseinrichtung 43 angeordnet, die über ein Kabel 44 mit einer nicht dargestellten Steuerungs- und Bilderzeugungselektronik in Verbindung steht und es gestattet, den Fokusbereich der Stoßwellen abzubilden. Vorzugsweise handelt es sich bei der Ultraschall-Ortungseinrichtung um einen an sich bekannten Ultraschall-Sektorscanner, der so angeordnet ist, daß eine die Mittelachse der Membran 1 und damit den Fokusbereich der Stoßwellen enthaltende kreissektorförmige Schicht des Körpers 38 des Patienten abtastbar ist.

Ein im wesentlichen gemäß Fig. 1 ausgeführter Prototyp einer erfindungsgemäßen Stoßwellenquelle besitzt eine Aluminium-Membran mit einem Außendurchmesser von 56 mm, einer Dicke von 0,3 mm und einer Länge von 60 mm. Die Spule des Prototyps ist aus einem Draht kreisförmigen Querschnitts mit einem Durchmesser von 0,5 mm auf einen Spulenträger aus Hartgewebe gewickelt, und weist drei parallelgeschaltete Wicklungen zu je 29 Windungen auf. Der Reflektor ist auf Grundlage einer Parabel zweiter Ordnung mit einem Halbparameter von 200 mm ausgebildet und besitzt an seinem dem Brennpunkt der Parabel zugewandten Ende einen Innendurchmesser von 168 mm und an seinem anderen Ende einen Innendurchmesser von 118 mm. Der Abstand des Brennpunktes von der Vorderkante des Reflektors beträgt 80 mm. Der Reflektor des Prototyps besteht aus Messing. Als Ausbreitungsmedium fand bei dem Prototypen Wasser Verwendung.

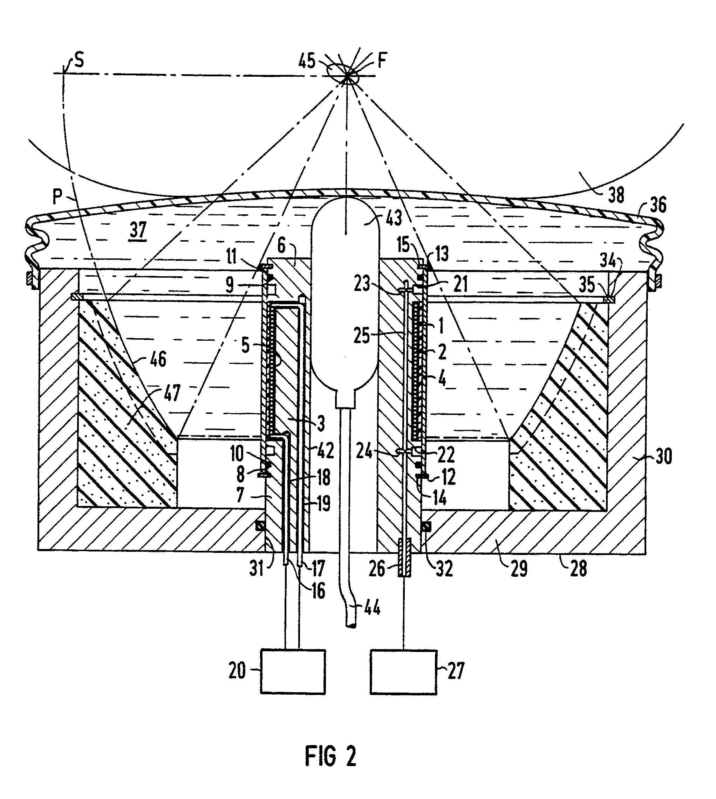

In der Fig. 2 ist eine weitere Ausführungsform der erfindungsgemäßen Stoßwellenquelle dargestellt, die insbesondere zur Beschallung pathologischer Gewebeveränderungen, z.B. eines im Körper 38 eines Patienten befindlichen Tumors 45, dient. Die Stoßwellenquelle gemäß Fig. 2 unterscheidet sich von dem zuvor beschriebenen Ausführungsbeispiel lediglich durch ein unterschiedliches Material des die Reflektorfläche 46 aufweisenden Reflektors 47, weshalb die übrigen Elemente der Stoßwellenquelle gemäß Fig. 2 die gleichen Bezugszeichen wie in Fig. 1 tragen.

Im Gegensatz zu dem zuvor beschriebenen Ausführungsbeispiel besteht im Falle der Fig. 2 der Reflektor 47 aus einem Material, das akustisch weicher als das Ausbreitungsmedium 37 ist. Wenn als Ausbreitungsmedium 37 Wasser vorgesehen ist, kommt als Material für den Reflektor 47 insbesondere ein Schaumstoff mit geschlossenen Poren wie z.B. Polyurethanschaum in Frage. Dabei genügt es, wenn dieses Material wenigstens im Bereich der Reflektorfläche 46 des Reflektors 47 wie strichliert angedeutet als ausreichend dicke Schicht vorhanden ist. Im Falle des dargestellten Ausführungsbeispieles besteht der Reflektor jedoch insgesamt aus dem Schaumstoff mit geschlossenen Poren.

In ihrer Funktionsweise unterscheidet sich die Stoßwellenquelle gemäß Fig. 2 von der zuvor beschriebenen insofern, als infolge des Umstandes, daß der Reflektor 47 aus einem Material besteht, das akustisch weicher als das Ausbreitungsmedium 37 ist, - hierunter versteht man, daß der akustische Wellenwiderstand dieses Materials kleiner als der des Übertragungsmediums ist, - Stoßwellen in Form von Unterdruckimpulsen erzeugt werden. Der Grund für diese unterschiedliche Funktionsweise liegt darin, daß bei Reflexionen an einer schallweichen Grenzschicht eine Phasenumkehr stattfindet (Reflexionsfaktor ist negativ). Eine Beschallung von Tumoren mit Stoßwellen in Form von Unterdruckimpulsen ist aus den bereits erläuterten Gründen einer Beschallung mit Stoßwellen in Form von Druckimpulsen vorzuziehen.

Im Falle des beschriebenen Ausführungsbeispieles ist lediglich eine einzige zylinderschraubenförmig gewickelte Spule 2 vorhanden. Es können aber auch mehrere solcher Spulen vorhanden sein, die beispielsweise axial aufeinanderfolgend auf den Spulen körper 3 gewickelt sind. In Abhängigkeit von der Anzahl der Spulen, die mit dem zur Erzeugung einer Stoßwelle dienenden Hochspannungsimpuls beaufschlagt werden, können dann Stoßwellen unterschiedlicher Intensität erzeugt werden.

Die Reflektorfläche 39 bzw. 46 muß nicht exakt rotationsparaboloidförmig ausgebildet sein, sondern kann auch durch Rotation eines Abschnittes eines Kreises, einer Ellipse oder dergleichen erhalten werden, sofern die Wirkung der so erhaltenen Reflektorfläche von der einer rotationsparaboloidförmigen nicht wesentlich abweicht.

## Ansprüche

1. Stoßwellenquelle zur Erzeugung von fokussierten Stoßwellen, aufweisend eine im wesentlichen hohlzylindrische Membran (1) aus einem elektrisch leitenden Werkstoff, eine innerhalb der Membran (1) angeordnete elektrische Spulenanordnung, welche zum stoßartigen Antrieb der Membran (1) mit einem Hochspannungsimpuls beaufschlagbar ist, einen die Membran (1) umgebenden konkaven Reflektor (33, 46), dessen Form wenigstens näherungsweise die eines Rotationsparaboloids ist und dessen Mittelachse im wesentlichen der Mittelachse der Membran (1) entspricht, und ein wenigstens den Raum zwischen der Membran (1) und dem Reflektor (33, 47) ausfüllendes akustisches Ausbreitungsmedium (37), **dadurch gekennzeichnet,** daß die Spulenanordnung wenigstens eine zylinderschraubenförmig gewickelte Spule (2) aufweist und daß die Membran (1) als dünnwandiges, glattes Rohr ausgebildet ist.

2. Stoßwellenquelle nach Anspruch 1, **dadurch gekennzeichnet,** daß die Spule (2) konzentrisch zur Membran (1) angeordnet ist.

3. Stoßwellenquelle nach Anspruch 1 oder 2, **dadurch gekennzeichnet,** daß die Membran (1) als nahtloses Rohr ausgeführt ist.

4. Stoßwellenquelle nach einem der Ansprüche 1 bis 3, **dadurch gekennzeichnet,** daß die Membran (1) eine konstante Dicke aufweist.

5. Stoßwellenquelle nach einem der Ansprüche 1 bis 4, **dadurch gekennzeichnet,** daß die Membran (1) wenigstens ein Material der Gruppe Aluminium, Kupfer, Silber oder Bronze enthält.

6. Stoßwellenquelle nach einem der Ansprüche 1 bis 5, **dadurch gekennzeichnet,** daß zwischen der Spule (2) und der Innenwand der Membran (1) Isoliermittel (4) vorgesehen sind.

7. Stoßwellenquelle nach einem der Ansprüche 1 bis 6, **dadurch gekennzeichnet,** daß der zwischen der Innenwand der Membran (1) und der Spule (2) bzw. den Isoliermitteln (4) befindliche Raum mit Unterdruck beaufschlagbar ist.

8. Stoßwellenquelle nach einem der Ansprüche 1 bis 7, **dadurch gekennzeichnet,** daß im Inneren der Spule (2) eine Ultraschall-Ortungseinrichtung (43) angeordnet ist, mittels derer der Fokusbereich (F) der Stoßwellen abbildbar ist.

9. Stoßwellenquelle nach einem der Ansprüche 1 bis 8, **dadurch gekennzeichnet,** daß die Spule (2) auf einen wenigstens im Bereich der Spule (2) zylindrisch ausgebildeten Spulenträger (3) aus einem elektrisch isolierenden Werkstoff gewickelt ist.

10. Stoßwellenquelle nach den Ansprüchen 8 und 9, **dadurch gekennzeichnet,** daß der Spulenträger (3) eine zentrale Bohrung (42) aufweist, in der die Ultraschall-Ortungseinrichtung (43) angeordnet ist.

11. Stoßwellenquelle nach einem der Ansprüche 1 bis 10, **dadurch gekennzeichnet,** daß der Reflektor (33) wenigstens im Bereich seiner Reflektorfläche (39) aus einem Material gebildet ist, das akustisch härter als das Ausbreitungsmedium (37) ist.

12. Stoßwellenquelle nach einem der Ansprüche 1 bis 10, **dadurch gekennzeichnet,** daß der Reflektor (47) wenigstens im Bereich seiner Reflektorfläche (46) aus einem Material gebildet ist, das akustisch weicher als das Ausbreitungsmedium (37) ist.

13. Stoßwellenquelle nach Anspruch 12, **dadurch gekennzeichnet,** daß der Reflektor (46) wenigstens im Bereich seiner Reflektorfläche (47) aus einem Schaumstoff mit geschlossenen Poren gebildet ist.

FIG 1

FIG 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE-A-3 727 692  (SIEMENS AG)<br>* Anspruch 1; Figur 1 *<br>--- | 1,8 | A 61 B  17/22<br>G 10 K  11/28 |
| A | FR-A-2 440 227  (O.N.E.R.A.)<br>* Ansprüche 1,5 *<br>------ | 1 | |

**RECHERCHIERTE SACHGEBIETE (Int. Cl.5)**

A 61 B
G 10 K
B 06 B

Der vorliegende Recherchenbericht wurde für alle Patentansprüche erstellt

| Recherchenort | Abschlußdatum der Recherche | Prüfer |
|---|---|---|
| BERLIN | 14-03-1990 | PAPA E.R. |